# EUROPÄISCHE PATENTSCHRIFT

(11) **EP 3 247 437 B1**
(45) Veröffentlichungstag und Bekanntmachung des Hinweises auf die Patenterteilung: **31.10.2018**
(21) Anmeldenummer: 17716248.4
(22) Anmeldetag: 10.04.2017
(51) Int. Cl.: A61M 16/00

(54) **BEATMUNGSGERÄT MIT VERBESSERTER SYNCHRONITÄT BEIM ÜBERGANG VON EXSPIRATORISCHEM ZU INSPIRATORISCHEM BETRIEB**
RESPIRATOR HAVING IMPROVED SYNCHRONICITY DURING THE TRANSITION FROM EXPIRATORY TO INSPIRATORY OPERATION
APPAREIL DE VENTILATION PRÉSENTANT UNE SYNCHRONICITÉ AMÉLIORÉE LORS DE LA TRANSITION D'UN FONCTIONNEMENT EXPIRATOIRE VERS UN FONCTIONNEMENT INSPIRATOIRE

(30) Priorität: 15.04.2016 DE 102016206442
(43) Veröffentlichungstag der Anmeldung: 29.11.2017
(73) Patentinhaber: Hamilton Medical AG, 7402 Bonaduz (CH)
(72) Erfinder: MEYER, Johannes Björn Thomas, 7000 Chur (CH); NOVOTNI, Dominik, 7000 Chur (CH)
(74) Vertreter: Ruttensperger Lachnit Trossin Gomoll
(86) Internationale Anmeldenummer: PCT/EP2017/058577
(87) Internationale Veröffentlichungsnummer: WO 2017/178440

(56) Entgegenhaltungen:
- EP-A1- 0 521 314
- EP-A2- 0 714 670
- DE-A1- 19 528 113
- DE-A1-102007 052 897
- DE-C1- 4 432 219
- US-B1- 6 439 229
- JOSEF X. BRUNNER ET AL: "Simple method to measure total expiratory time constant based on the passive expiratory flow-volume curve :", CRITICAL CARE MEDICINE., Bd. 23, Nr. 6, 1. Juni 1995 (1995-06-01), Seiten 1117-1122, XP055378938, US ISSN: 0090-3493, DOI: 10.1097/00003246-199506000-00019 in der Anmeldung erwähnt

## Beschreibung

Die vorliegende Erfindung betrifft ein Beatmungsgerät zur wenigstens unterstützenden Beatmung von im gesunden Zustand atmenden Lebewesen, wobei das Beatmungsgerät umfasst:
- eine Anschlussformation zur Verbindung mit einem Atemgasvorrat,
- eine bei inspiratorischem Betrieb des Beatmungsgeräts frisches inspiratorisches Atemgas von der Anschlussformation zu einer Patientenschnittstelle hin führende und bei exspiratorischem Betrieb verstoffwechseltes exspiratorisches Atemgas von der Patientenschnittstelle weg führende Atemgas-Leitungsanordnung,
- eine Druckveränderungsanordnung zur Veränderung des Drucks von Atemgas in der Atemgas-Leitungsanordnung,
- einen Flusssensor, welcher dazu ausgebildet und angeordnet ist, ein einen Atemgasstrom wenigstens von exspiratorischem Atemgas in der Atemgas-Leitungsanordnung repräsentierendes Flusssignal bereitzustellen,
- eine den Betrieb des Beatmungsgeräts steuernde Steuereinrichtung, welche dazu ausgebildet ist, einen Übergang von exspiratorischem Betrieb zu inspiratorischem Betrieb des Beatmungsgeräts dann zu triggern, wenn eine Zunahme der Steilheit eines Flusssignalverlaufs, welcher zeitlich nacheinander bereitgestellte Flusssignale wiedergibt, einen Steilheit-Änderungsschwellenwert übersteigt.

Ein derartiges gattungsgemäßes Beatmungsgerät ist aus der EP 0 521 314 A1 bekannt. Diese Druckschrift lehrt, ein Beatmungsgerät zwischen einer Exspirationsphase und einer Inspirationsphase dann umzusteuern, wenn die während der Beatmungsphase ermittelte Atemströmungskurve eine signifikante Zunahme ihrer Steilheit zeigt. Ob eine solche signifikante Zunahme vorliegt oder nicht, wird anhand eines Vergleichs einer auftretenden Steilheitszunahme mit einem vorbestimmten Steilheit-Änderungsschwellenwert bestimmt. Diese Lehre stellt eine Verbesserung anderer, ebenfalls aus dem Stand der Technik bekannter Triggerkriterien für einen Übergang von exspiratorischem zu inspiratorischem Betrieb des Beatmungsgeräts dar.

Ein sehr einfaches Triggerkriterium stellt der Basisniveaudurchgang, etwa ein Nulldurchgang oder ein Durchgang durch ein von Null verschiedenes Basisniveau, der Atemströmungskurve, also des zeitlichen Flusssignalverlaufs, dar. Dieses Triggerkriterium ist beispielsweise aus der US 3,834,382 bekannt. Nachteilig an diesem Kriterium ist, dass es im Wesentlichen nur bei Patienten mit gesundem Atemsystem zufriedenstellend funktioniert, wohingegen während der künstlichen Beatmung von chronisch obstruktiven Patienten (COPD-Patienten) für den Patienten unangenehme Asynchronitäten auftreten können, wenn der Patient während der künstlichen Beatmung Spontanatmung zeigt.

Darüber hinaus ist das Triggerkriterium eines Basisniveaudurchgangs des Flusssignals empfindlich gegen leckagebedingte Effekte, die bei künstlicher Beatmung auftreten können, beispielsweise weil eine Beatmungsmaske als eine mögliche Patientenschnittstelle nicht vollständig mit der Gesichtspartie um Nase und Mund des zu beatmenden Patienten abschließt. Auch andere Ursachen können Leckage-Ströme und somit leckagebedingte Effekte während der künstlichen Beatmung bewirken.

Zur Vermeidung von leckagebedingten Fehlern bei einer ETS-basierten Triggerung lehrt die DE 44 32 219 C1, eine Atemgasfluss-Triggerschwelle für einen Übergang von exspiratorischem Betrieb zu inspiratorischem Betrieb um einen Zusatzbetrag zu erhöhen, der gebildet ist aus dem Verhältnis zwischen dem gemittelten Atemdruck während der Inspiration und der Summe der gemittelten Atemdrücke während der Inspiration und Exspiration, multipliziert mit der Differenz der gemittelten Werte der Atemströmungen während der Inspiration und während der Exspiration.

Auch die aus der DE 44 32 219 C1 bekannte ETS-basierte technische Lehre kann jedoch für Patienten mit pathologischen Atemorganen, wie etwa COPD-Patienten, zu unerwünschten Asynchronitäten während der künstlichen Beatmung führen.

Aus der US 6,439,229 B1 ist es außerdem bekannt, für ein sogenanntes "Cycling", also für einen Übergang von inspiratorischem zu exspiratorischem Betrieb des Beatmungsgeräts, ETS-basierte Entscheidungsschwellen abhängig von der exspiratorischen Zeitkonstante des jeweils beatmenden Patienten zu verändern.

Mit ETS ist die sogenannte "Expiratory Trigger Sensitivity" bezeichnet. Dabei handelt es sich um einen Prozentsatz des während eines Inspirationsvorgangs auftretenden maximalen Atemgasflusses, welcher als Cycling-Schwellenwert dient. Dann, wenn der Atemgasfluss während der Inspirationsphase auf den vorbestimmten Cycling-Schwellenwert abgefallen ist, wird das Beatmungsgerät vom inspiratorischen Betrieb auf den exspiratorischen Betrieb umgesteuert. Für gesunde Patienten liegt der ETS-basierte Cycling-Schwellenwert häufig bei etwa 25 % des während der Inspirationsphase auftretenden maximalen Atemgasflusses.

Die US 6,439,229 B1 lehrt nun, abhängig von der ermittelten exspiratorischen Zeitkonstante des jeweils beatmeten Patienten einen Bereich von möglichen Cycling-Schwellenwerten vorauszuwählen und abhängig von dem Betrag eines als "supraplateau-pressure" bezeichneten Drucküberschwingers im Atemgasdruckverlauf am Ende der druckunterstützten Inspirationsphase innerhalb des vorausgewählten Prozentbereiches einen konkreten Prozentwert vom maximal auftretenden Inspirationsatemgasfluss als Cycling-Schwellenwert auszuwählen.

Mit der aus der US 6,439,229 B1 bekannten technischen Lehre kann zwar die Synchronität der künstlichen Beatmung durch ein Beatmungsgerät mit einem spontan atmenden Patienten verbessert werden, wegen des ETS-basierten Steuerverhaltens des bekannten Beatmungsgeräts ist dieses jedoch anfällig für häufig auftretende Leckage-Strömungen, was den Synchronitätsvorteil durch den anhand der exspiratorischen Zeitkonstante und des beschriebenen Druckverhaltens angepassten Cycling-Schwellenwert wieder zunichtemachen kann.

Mit der aus der gattungsgemäßen EP 0 521 314 A1 bekannten technischen Lehre wird eine, verglichen mit ETS-basierten Trigger- und Cycling-Schwellenwerten verringerte Anfälligkeit gegenüber Leckage-Strömungen erhalten, da es für ein Triggern eines Überganges vom exspiratorischen zum inspiratorischen Betrieb nicht mehr auf einen konkreten Wert oder ein konkretes Werteverhältnis des Atemgasflusses ankommt, sondern auf die Gestalt des zeitlichen Atemgas-Flusssignalverlaufs. Diese Gestalt bleibt auch bei auftretender Leckage in gewissen Grenzen erhalten. Auch kann ein gemäß der Lehre der EP 0 521 314 A1 arbeitendes Beatmungsgerät chronisch obstruktive Patienten, verglichen mit anderen, etwa ETS-basiert arbeitenden Beatmungsgeräten, mit verbesserter Synchronität beatmen. Allerdings ist COPD nur eine mögliche auftretende pathologische Atemsystemveränderung. Patienten mit anderen ebenso auftretenden Atemsystemveränderungen, welche zu einem von einem gesunden Atemverhalten abweichenden Atemverhalten führen, können mit dem aus der EP 0 521 314 A1 bekannten technischen Lehre sogar mit verschlechterter Synchronität beatmet werden, verglichen mit ETS-basiert arbeitenden Beatmungsgeräten.

Darüber hinaus ist dann, wenn sowohl Patienten mit unbeeinträchtigtem Atemsystem als auch COPD-Patienten durch ein und dasselbe gemäß der EP 0 521 314 A1 arbeitende Beatmungsgerät beatmet werden sollen, der dabei verwendete vorbestimmte Steilheit-Änderungsschwellenwert stets ein Kompromiss, der für alle Patienten anwendbar sein soll und daher im Einzelfall zu Asynchronitäten in der Beatmung führen kann, weil der jeweils zu beatmende Patient auf den dem vorbestimmten Steilheit-Änderungsschwellenwert zugrunde liegenden Fall nur bedingt zutrifft.

Es ist daher Aufgabe der vorliegenden Erfindung, das eingangs genannte Beatmungsgerät dahingehend zu verbessern, dass mit ihm Patienten ungeachtet des pathologischen Zustands ihres Atemsystems mit verbesserter Synchronität beatmet werden können.

Diese Aufgabe löst die vorliegende Erfindung durch ein gattungsgemäßes Beatmungsgerät, bei welchem die Steuereinrichtung weiter dazu ausgebildet ist, den Steilheit-Änderungsschwellenwert abhängig von einer exspiratorischen Zeitkonstante des jeweils zu beatmenden Patienten zu ermitteln und den Übergang von exspiratorischem Betrieb zu inspiratorischem Betrieb des Beatmungsgeräts dann zu triggern, wenn die Zunahme der Steilheit des Flusssignalverlaufs den nach Maßgabe der exspiratorischen Zeitkonstante ermittelten Steilheit-Änderungsschwellenwert übersteigt.

Durch die Ermittlung, gegebenenfalls Änderung und damit Anpassung des Steilheit-Änderungsschwellenwertes abhängig von der exspiratorischen Zeitkonstante des jeweils zu beatmenden Patienten kann der Übergang von exspiratorischem Betrieb zu inspiratorischem Betrieb des Beatmungsgerätes an den pathologischen Zustand des Atemsystems des jeweiligen Patienten nahezu optimal angepasst werden, da die exspiratorische Zeitkonstante einer Person einen für die künstliche Beatmung eines Patienten hochrelevanten Parameter darstellt.

Durch die Veränderung des Steilheit-Änderungsschwellenwertes abhängig von der exspiratorischen Zeitkonstante des jeweils zu beatmenden Patienten kann so eine Anpassung des Betriebs des Beatmungsgerätes zumindest beim Übergang von exspiratorischem zu inspiratorischem Betrieb an beliebige pathologische Zustände der Atemsysteme von Patienten erfolgen.

Es sei klargestellt, dass mit dem Begriff "Triggern" in der vorliegenden Anmeldung lediglich das Auslösen eines Überganges von exspiratorischem Betrieb zu inspiratorischem Betrieb des Beatmungsgeräts bezeichnet ist. Für das Auslösen eines Übergangs in entgegengesetzter Richtung von inspiratorischem zu exspiratorischem Betrieb wird dagegen der Begriff "Cycling" verwendet.

Mit der erfindungsgemäß ausgebildeten Steuereinrichtung können nicht nur COPD-Patienten, welche verglichen mit Patienten mit gesunden Atemsystemen eine überdurchschnittlich hohe exspiratorische Zeitkonstante aufweisen, mit hervorragender Synchronität beatmet werden, sondern auch ARDS-Patienten, deren exspiratorische Zeitkonstante verglichen mit Patienten mit gesunden Atemsystemen überdurchschnittlich kurz ist.

Eine bei dem vorliegend diskutierten Übergang von exspiratorischem zu inspiratorischem Betrieb häufig auftretende Asynchronität ist das sogenannte "Autotriggern", bei welchem das Beatmungsgerät aufgrund entsprechend voreingetellter Entscheidungskriterien einen Inspirationsvorgang auslöst, ohne dass der Patient tatsächlich einatmet oder Anstrengungen für einen Inspiration unternimmt. Es entsteht dann die Situation, dass das Beatmungsgerät dem Patienten, der eigentlich noch ausatmen möchte oder wenigstens im Begriff ist, auszuatmen, gegen dessen Atemanstrengung Atemgas einführt. Dies kann bei Patienten trotz Sedierung heftige Reaktionen auslösen, die auch für die weitere künstliche Beatmung unvorteilhaft sind.

COPD, was landläufig auch als "Asthma" oder "Raucherlunge" bezeichnet wird, führt aufgrund des obstruktiven Atemverhaltens zu verhältnismäßig stetigen Atemgas-Flusssignalverläufen, an denen eine betragsmäßige Änderung ihrer Steilheit relativ einfach und sicher feststellbar ist, sodass auch das bereits bekannte gattungsgemäße Beatmungsgerät für COPD-Patienten verhältnismäßig gute Ergebnisse liefert.

ARDS-Patienten weisen ein restruktives Atemsystem auf, was landläufig als "harte" Lunge bezeichnet wird. Die Atmung erfolgt hier häufig stoßartig, was während einer Exspirationsphase zu einer oder mehreren Unstetigkeiten des Atemgas-Flusssignalverlaufs in Form von Knicken führen kann. Diese Knickstellen stellen betragsmäßige Änderungen der Steilheit des Flusssignals dar, welche bei unvorteilhaft gewähltem Steilheit-Änderungsschwellenwert ein Autotriggern auslösen können.

Die vorliegende Erfindung hilft, auch ARDS-Patienten mit dem erfindungsgemäß verbesserten Beatmungsgerät mit verbesserter Synchronität beatmen zu können.

Wegen des geschilderten Zusammenhangs ist es vorteilhaft, ARDS-Patienten, deren exspiratorische Zeitkonstante kleiner als jene von Patienten mit gesundem Atemsystem ist, mit einem betragsmäßig höheren Steilheit-Änderungsschwellenwert zu beatmen, um ARDS-typische Unstetigkeiten im Flusssignalverlauf während einer Exspirationsphase durch den Steilheit-Änderungsschwellenwert nicht zu erfassen und korrekterweise erst eine betragsmäßig größere Änderung der Steilheit des Flusssignalverlaufs am Ende der Exspirationsphase als Triggersignal heranzuziehen, wenn tatsächlich auch in Übereinstimmung mit dem Patientenverhalten ein Übergang von exspiratorischem zu inspiratorischem Betrieb stattfinden soll.

Ebenso können COPD-Patienten, deren exspiratorische Zeitkonstante größer als jene von Patienten mit gesundem Atemsystem ist, mit einem Steilheit-Änderungsschwellenwert beatmet werden, der betragsmäßig geringer ist als der von ARDS-Patienten und geringer ist als der von Patienten mit gesundem Atemsystem.

Daher ist die Steuereinrichtung vorzugsweise dazu ausgebildet, den Betrag des Steilheit-Änderungsschwellenwerts mit zunehmender exspiratorischer Zeitkonstante zu verringern und umgekehrt.

Bevorzugt ist die Steuereinrichtung zur Vermeidung von unerwünschten Fehltriggerungen dazu ausgebildet, nur solche Änderungen der Steilheit des Flusssignalverlaufs auf das Vorliegen des Überschreiten des Steilheit-Änderungsschwellenwerts zu überprüfen, durch welche die Steigung des Flusssignalverlaufs zunimmt, also der Flusssignalverlauf progressiv ansteigt. Der Steilheit-Änderungsschwellenwert ist dann bevorzugt ein Steilheit-Zunahmeschwellenwert, anzuwenden in ansteigenden Flussignalverlaufsabschnitten, nicht dagegen in abfallenden.

Weiterhin sei an dieser Stelle klargestellt, dass das in der vorliegenden Anmeldung diskutierte Triggerkriterium nicht das einzige Triggerkriterium ist, gemäß welchem die Steuereinrichtung den Betrieb der Beatmungsvorrichtung von exspiratorisch auf inspiratorisch ändert oder ändern kann. Die Steuereinrichtung weist bevorzugt eine Mehrzahl, etwa mehr als 25, Triggerkriterien auf, von welchen jedes im Falle seines Vorliegens die Steuereinrichtung veranlasst, den Betrieb der Beatmungsvorrichtung von exspiratorisch auf inspiratorisch umzusteuern.

Grundsätzlich kann gemäß einem einfachen Ausführungsbeispiel der vorliegenden Erfindung vorgesehen sein, dass die Steuereinrichtung mit einer Eingabevorrichtung, etwa einer Tastatur und dergleichen, zusammenwirkt, durch die die Eingabe einer für den jeweils zu beatmenden Patienten zutreffenden exspiratorischen Zeitkonstante möglich ist.

Bereits mit dem erwähnten Flusssensor ist es grundsätzlich möglich, die exspiratorische Zeitkonstante eines zu beatmenden Patienten zu ermitteln, weshalb die Steuereinrichtung bevorzugt dazu ausgebildet ist, die exspiratorische Zeitkonstante in einer jeweiligen Beatmungsanwendung zu ermitteln. Der Flusssensor erfasst den Atemgasstrom bevorzugt als Volumenstrom. Eine zusätzliche oder alternative Erfassung als Massenstrom ist ebenfalls möglich.

Dabei kann daran gedacht sein, die Zeitkonstante einmal zu Beginn der Beatmungsanwendung für die gesamte restliche Dauer der Beatmungsanwendung zu ermitteln und mit dem ermittelten Wert die Beatmungsanwendung durchzuführen.

Alternativ kann daran gedacht sein, die exspiratorische Zeitkonstante in vorbestimmten Zeitabständen während einer Beatmungsanwendung immer wieder zu ermitteln und die Beatmungsanwendung mit dem jeweils zuletzt ermittelten Zeitkonstantenwert fortzusetzen. Eine besonders genaue und damit vorteilhaft besonders synchrone Beatmung eines Patienten kann dadurch erfolgen, dass die Steuereinrichtung dazu ausgebildet ist, die exspiratorische Zeitkonstante atemzugsweise zu ermitteln.

Grundsätzlich sind zahlreiche Möglichkeiten bekannt, eine exspiratorische Zeitkonstante eines Patienten automatisiert zu ermitteln. Besonders vorteilhafte Methoden hierzu sind offenbart in Josef X. Brunner et al: "Simple method to measure total expiratory time constant based on the passive expiratory flow-volume curve", in: CRITICAL CARE MEDICINE, 1995, Vol. 23, No. 6, Seiten 1117 ff., auf deren Inhalt hier verwiesen wird.

Beispielsweise kann die Steuereinrichtung dazu ausgebildet sein, die exspiratorische Zeitkonstante nach Maßgabe des Verhältnisses zwischen dem während eines Exspirationsvorgangs ausgeatmeten Volumen und dem dabei aufgetretenen maximalen exspiratorischen Volumenstrom, bevorzugt iterativ numerisch, oder/und nach Maßgabe der bei einem Atemhub auftretenden Resistance und Compliance zu ermitteln. Die Resistance und die Compliance getrennt zu ermitteln und daraus aus Multiplikation die exspiratorische Zeitkonstante zu errechnen, ist möglich, aber weniger bevorzugt als die Zeitkonstante aus dem Verhältnis von ausgeatmetem Volumen und dabei aufgetretenem maximalen exspiratorischen Volumenstrom zu berechnen. Hier existieren iterative nummerische Berechnungsmethoden, welche bereits nach wenigen Iterationsschritten zu einem zutreffenden Wert konvergieren, der sich von der tatsächlichen Zeitkonstante für den jeweiligen Beatmungsfall nur in vernachlässigbarem Umfang unterscheidet.

Der Begriff "Flusssignalverlauf" soll nicht dahingehend verstanden werden, dass der vollständige zeitliche Verlauf des vom Flusssensor bereitgestellten Flusssignals aufgezeichnet und bereitgestellt werden muss. Es reicht zur Erzielung der Vorteile der vorliegenden Erfindung aus, wenn der Flusssignalverlauf über eine so lange Zeitspanne vorliegt, dass daraus die Änderung einer Steilheit des Flusssignalverlaufs ermittelt werden kann. Auch soll der Begriff "Flusssignalverlauf" nicht dahingehend missverstanden werden, dass zwingend ein kontinuierlicher Verlauf zur Ermittlung einer Änderung von dessen Steilheit benötigt wird. Auch eine Folge von zeitlich aufeinanderfolgenden diskreten Flusssignalwerten stellt einen Flusssignalverlauf im Sinne der vorliegenden Anmeldung dar. Dabei reichen beispielsweise drei zeitlich nacheinander ermittelte Flusssignalwerte aus, um eine Änderung der Steilheit dieses kurzen Flusssignalverlaufs zu ermitteln und auszuwerten. So kann zwischen erstem und zweitem Flusssignalwert mittels eines Differenzenquotienten eine Steigung des Flusssignalverlaufs zwischen diesen beiden Stützpunkten ermittelt werden und ebenso zwischen zweitem und drittem Flusssignalwert, wiederum durch einen Differenzenquotienten. Die so ermittelten Steigungen einmal zwischen erstem und zweitem Flusssignalwert und ein weiteres Mal zwischen zweitem und drittem Flusssignalwert können miteinander verglichen werden, beispielsweise durch Subtraktion, wobei der Unterschiedswert, der ein Maß für die Änderung der Steigung beziehungsweise Steilheit des Flusssignalverlaufs darstellt, mit dem Steilheit-Änderungsschwellenwert des Flusssignalverlaufs verglichen werden kann. Abhängig vom Ausgang eines solchen Vergleichs mit dem Steilheit-Änderungsschwellenwert kann dann das Triggern veranlasst werden oder nicht.

Wenn in der vorliegenden Anmeldung von zeitlich nacheinander ermittelten oder bereitgestellten Flusssignalwerten die Rede ist, so bedeutet dies bevorzugt, aber nicht notwendigerweise, unmittelbar nacheinander ermittelt oder bereitgestellt.

Selbstverständlich sind weitere Möglichkeiten zur Ermittlung der Änderung der Steilheit eines Flusssignalverlaufs bekannt. Beispielsweise kann der Flusssignalverlauf wenigstens abschnittsweise durch eine Funktion angenähert werden, welche Näherungsfunktion nach der Zeit zweimal abgeleitet werden kann. Die zweite Ableitung einer Funktion ist bekanntermaßen ein Maß für die Änderung der Steilheit einer Funktion oder eines Werteverlaufs.

Zur zielführenden Veränderung des Steilheit-Änderungsschwellenwertes auf Grundlage der exspiratorischen Zeitkonstante des zu beatmenden Patienten kann gemäß einer vorteilhaften Weiterbildung der vorliegenden Erfindung vorgesehen sein, dass das Beatmungsgerät einen von der Steuereinrichtung wenigstens lesbaren Datenspeicher aufweist, in welchem ein Zusammenhang zwischen dem Betrag des Steilheit-Änderungsschwellenwerts und der exspiratorischen Zeitkonstante oder ein Zusammenhang zwischen einem Änderungswert des Betrags des Steilheit-Änderungsschwellenwerts und der exspiratorischen Zeitkonstante hinterlegt ist. Der Zusammenhang kann über den Betrag des Steilheit-Änderungsschwellenwerts hinaus auch dessen Vorzeichen betreffen.

Der Zusammenhang kann in Form eines tabellarischen oder graphischen Kennfelds hinterlegt sein. Bevorzugt ist im Datenspeicher ein funktioneller Zusammenhang zwischen dem Betrag des Steilheit-Änderungsschwellenwertes und der exspiratorischen Zeitkonstante oder ein funktioneller Zusammenhang zwischen einem Änderungswert des Betrages des Steilheit-Änderungsschwellenwertes und der exspiratorischen Zeitkonstante hinterlegt - je nachdem ob der Steilheit-Änderungsschwellenwert absolut ermittelt oder lediglich ein auf den zuvor verwendeten Steilheit-Änderungsschwellenwert anzuwendender Änderungsbetrag ermittelt werden soll, ob also die Änderung des Steilheit-Änderungsschwellenwertes absolut oder inkrementell erfolgen soll. Beides ist grundsätzlich möglich. Die Steuereinrichtung ist dann bevorzugt dazu ausgebildet, einen anzuwendenden Steilheit-Änderungsschwellenwert ausgehend von der exspiratorischen Zeitkonstante anhand des funktionellen Zusammenhangs zu ermitteln. Somit braucht keine Interpolation oder Extrapolation von Steilheit-Änderungsschwellenwerten berechnet werden, sondern es kann unmittelbar der jeweils für einen Zeitkonstantenwert zutreffende Steilheit-Änderungsschwellenwert ermittelt werden, gegebenenfalls unter Anwendung des Zwischenschrittes der Ermittlung eines Änderungsinkrements und Anwendung des Änderungsinkrements auf den zuletzt gültigen Steilheit-Änderungsschwellenwert.

Vorzugsweise weist das Beatmungsgerät einen von der Steuereinrichtung beschreibbaren Datenspeicher auf, in welchem vom Flusssensor bereitgestellte Flusssignalwerte gespeichert werden können, um aus den Werten einen zeitlichen Flusssignalverlauf zu ermitteln. Dies kann der oben bezeichnete Datenspeicher sein.

Häufig ist das vom Flusssensor erhaltene Flusssignal rauschbehaftet und zu einer unmittelbaren Auswertung daher suboptimal geeignet. Daher kann weiter vorgesehen sein, dass die Steuereinrichtung dazu ausgebildet ist, das vom Flusssensor bereitgestellte Flusssignal zu glätten, etwa durch Tiefpassfilterung oder durch gleitende Mittelwertbildung. Die gleitende Mittelwertbildung kann dabei die letzten fünf bis sechs Flusssignalwerte berücksichtigen, gegebenenfalls gewichtet. Eine Glättung des zeitlichen Flusssignalverlaufs ist vor allem dann vorteilhaft, wenn die Änderung der Steilheit des Flusssignalverlaufs durch Differenzieren einer an den Flusssignalverlauf wenigstens abschnittsweise angenäherten Näherungsfunktion erfolgen soll, da eine Näherungsfunktion für einen geglätteten Signalverlauf mit geringem Fehler ermittelbar ist. Bevorzugt erfolgt die Ermittlung der exspiratorischen Zeitkonstante auf Grundlage des geglätteten Flusssignalverlaufs.

Um ein unerwünschtes Triggern aufgrund von Steilheits-Änderungen des Flusssignalverlaufs in der Anfangsphase des Exspirationsbetriebs zu vermeiden, ist die Steuereinrichtung gemäß einer vorteilhaften Weiterbildung der vorliegenden Erfindung dazu ausgebildet, den Übergang von exspiratorischem Betrieb zu inspiratorischem Betrieb des Beatmungsgeräts nicht vor Ablauf einer vorbestimmten Zeitspanne nach einem vorgegebenen charakteristischen Ereignis, wie etwa ein Beginn des exspiratorischen Betriebs oder ein Basisniveaudurchgang des Flusssignals, zu triggern. Der Basisniveaudurchgang des Flusssignals kann ein Durchgang durch ein Nullniveau oder durch ein von Null verschiedenes Basisniveau sein.

Zu Beginn des Exspirationsbetriebs wird üblicherweise ein Inspirationsventil des Beatmungsgeräts geschlossen und ein Exspirationsventil geöffnet, wodurch es am Patienten zu einer Richtungsumkehrung des Atemgasstroms kommt. Das Flusssignal ändert dabei das Vorzeichen, fällt also zunächst betragsmäßig stark ab und steigt dann mit umgekehrten Vorzeichen betragsmäßig stark an. Ausgehend von dem dann auftretenden maximalen Atemgasfluss fällt das Flusssignal betragsmäßig mit fortschreitender Exspiration wieder ab. Es kommt daher in der Regel zu Beginn einer Exspirationsphase zu einer Änderung der Steilheit des Flusssignalverlaufs, die jedoch gerade keinen Übergang von exspiratorischem zu inspiratorischem Betrieb triggern soll. Dies wird mit der zuvor geschilderten Maßnahme verhindert.

Auch dabei kann daran gedacht sein, die vorbestimmte Zeitspanne, innerhalb der ab Eintritt des charakteristischen Ereignisses kein Übergang getriggert werden soll, nach Maßgabe der exspiratorischen Zeitkonstante zu ermitteln, da bei COPD-Patienten die Exspirationsphase länger dauert als bei Patienten mit gesundem Atemsystem und bei ARDS-Patienten die Exspirationsphase kürzer ist als bei Patienten mit gesundem Atemsystem.

Daher kann die vorbestimmte Zeitspanne bei ARDS-Patienten kürzer gewählt sein als bei Patienten mit gesundem Atemsystem und deren Zeitspanne wiederum kürzer als jene von COPD-Patienten. Die vorbestimmte Zeitspanne kann daher beispielsweise proportional zur exspiratorischen Zeitkonstante gewählt sein.

Das erfindungsgemäße Beatmungsgerät ist bevorzugt zur künstlichen Beatmung im PSV-Modus (PSV = "Pressure Support Ventilation") ausgebildet.

Die vorliegende Erfindung wird nachfolgend anhand der beiliegenden Zeichnungen näher erläutert. Es stellt dar:
- Figur 1: eine schematische Darstellung einer erfindungsgemäßen, zur künstlichen Beatmung eines Patienten hergerichteten Beatmungsvorrichtung,
- Figur 2a: einen beispielhaften zeitlichen Verlauf eines Flusssignals eines COPD-Patienten,
- Figur 2b: eine beispielhafte graphische Darstellung von im zeitlichen Flusssignalverlauf von Fig. 2a auftretenden Zunahmen der Steilheit des Flusssignalverlaufs mit anwendbarem Steilheit-Änderungsschwellenwert zur Triggerung eines Inspirationsvorgangs,
- Figur 3a: einen beispielhaften zeitlichen Verlauf eines Flusssignals eines ARDS-Patienten und
- Figur 3b: eine beispielhafte graphische Darstellung von im zeitlichen Flusssignalverlauf von Fig. 3a auftretenden Zunahmen der Steilheit des Flusssignalverlaufs mit anwendbarem Steilheit-Änderungsschwellenwert zur Triggerung eines Inspirationsvorgangs.

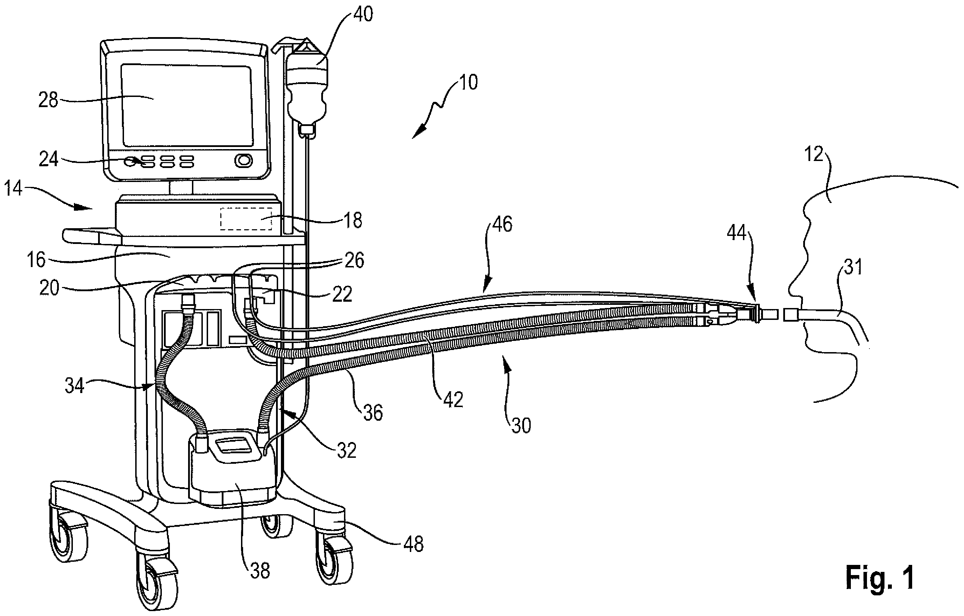

In Figur 1 ist eine erfindungsgemäße Ausführungsform einer Beatmungsvorrichtung allgemein mit 10 bezeichnet. Die Beatmungsvorrichtung 10 dient im dargestellten Beispiel zur künstlichen Beatmung eines humanen Patienten 12 im PSV-Modus.

Die Beatmungsvorrichtung 10 weist ein Gehäuse 14 auf, in dem - von außen wegen des blickdichten Gehäusematerials nicht erkennbar - eine Druckveränderungsanordnung 16 und eine Steuereinrichtung 18 aufgenommen sein können.

Die Druckveränderungsanordnung 16 ist in an sich bekannter Weise aufgebaut und kann eine Pumpe, einen Verdichter, ein Gebläse, einen Druckbehälter, ein Reduzierventil und dergleichen aufweisen. Weiter weist die Beatmungsvorrichtung 10 in an sich bekannter Weise ein Inspirationsventil 20 und ein Exspirationsventil 22 auf.

Die Steuereinrichtung 18 ist üblicherweise als Computer oder Mikroprozessor realisiert. Sie umfasst eine in Figur 1 nicht dargestellte Speichereinrichtung, um für den Betrieb der Beatmungsvorrichtung 10 notwendige Daten speichern und erforderlichenfalls aufrufen zu können. Die Speichereinrichtung kann bei Netzwerkbetrieb auch außerhalb des Gehäuses 14 gelegen und durch eine Datenübertragungsverbindung mit der Steuereinrichtung 18 verbunden sein. Die Datenübertragungsverbindung kann durch eine Kabel- oder eine Funkstrecke gebildet sein. Um jedoch zu verhindern, dass Störungen der Datenübertragungsverbindung sich auf den Betrieb der Beatmungsvorrichtung 10 auswirken können, ist die Speichereinrichtung bevorzugt in die Steuereinrichtung 18 integriert oder wenigstens im selben Gehäuse 14 wie diese aufgenommen.

Zur Eingabe von Daten in die Beatmungsvorrichtung 10 bzw. genauer in die Steuereinrichtung 18 kann die Beatmungsvorrichtung 10 einen Dateneingang 24 aufweisen, welcher in dem in Figur 1 dargestellten Beispiel durch eine Tastatur repräsentiert ist. Wie weiter unten noch erläutert werden wird, ist die Tastatur nicht der einzige Dateneingang der Steuereinrichtung 18. Tatsächlich kann die Steuereinrichtung 18 Daten über verschiedene Dateneingänge erhalten, etwa über eine Netzwerkleitung, eine Funkstrecke oder über Sensoranschlüsse 26, auf die weiter unten im Einzelnen eingegangen wird.

Zur Ausgabe von Daten an den behandelnden Therapeuten kann die Beatmungsvorrichtung 10 ein Ausgabegerät 28 aufweisen, im dargestellten Beispiel einen Bildschirm.

Zur künstlichen Beatmung ist der Patient 12 mit der Beatmungsvorrichtung 10, genauer mit der Druckveränderungsanordnung 16 im Gehäuse 14, über eine Atemgas-Leitungsanordnung 30 verbunden. Der Patient 12 ist hierfür intubiert. Der Tubus 31 bildet eine Patientenschnittstelle des Beatmungsgeräts 10. Alternativ kann die Patientenschnittstelle eine Nase und Mund überdeckende Maske umfassen.

Das Beatmungsgerät 10 weist überdies eine in Figur 1 nicht dargestellte Anschlussformation zur Verbindung mit einem Atemgasvorrat auf. Diese Anschlussformation kann in einem einfachen Fall ein Ansaugrohr sein, durch welches Umgebungsluft - gewünschtenfalls unter Zwischenanordnung eines Filters - aus der unmittelbaren Umgebung des Beatmungsgeräts 10 in die Atemgas-Leitungsanordnung 30 angesaugt werden kann. Die Anschlussformation kann auch eine Strömungsleitungkupplung sein, mittels welcher das Beatmungsgerät mit einem Gasvorrat - sei es Luft oder sei es Sauerstoff - verbindbar ist. Der Gasvorrat kann beispielsweise ein Gasbehälter sein oder ein Sammelvorrat sein, wie er in Kliniken als Teil der Haustechnik installiert ist.

Die Atemgas-Leitungsanordnung 30 weist einen Inspirationsschlauch 32 auf, über welchen frisches Atemgas von der Druckveränderungsanordnung 16 in die Lunge des Patienten 12 geleitet werden kann. Der Inspirationsschlauch 32 kann unterbrochen sein und einen ersten Inspirationsschlauch 34 und einen zweiten Inspirationsschlauch 36 aufweisen, zwischen welchen eine Konditionierungseinrichtung 38 zur gezielten Befeuchtung und gegebenenfalls auch Temperierung des dem Patienten 12 zugeführten frischen Atemgases vorgesehen sein kann. Die Konditionierungseinrichtung 38 kann mit einem externen Flüssigkeitsvorrat 40 verbunden sein, über den Wasser zur Befeuchtung oder auch ein Medikament, etwa zur Entzündungshemmung oder zur Erweiterung der Atemwege, der Konditionierungseinrichtung 38 zugeführt werden kann. Bei einem Einsatz der vorliegenden Beatmungsvorrichtung 10 als Anästhesie-Beatmungsvorrichtung können so volatile Anästhetika kontrolliert über die Beatmungsvorrichtung 10 an den Patienten 12 abgegeben werden. Die Konditionierungseinrichtung 38 sorgt dafür, dass das frische Atemgas dem Patienten 12 mit einem vorbestimmten Feuchtegehalt, gegebenenfalls unter Zugabe eines Medikamenten-Aerosols und mit einer vorbestimmten Temperatur zugeleitet wird.

Die Atemgas-Leitungsanordnung 30 weist neben dem bereits erwähnten Inspirationsventil 20 und Exspirationsventil 22 weiter einen Exspirationsschlauch 42 auf, über welchen verstoffwechseltes Atemgas aus der Lunge des Patienten 12 in die Atmosphäre abgeblasen wird.

Der Inspirationsschlauch 32 ist mit dem Inspirationsventil 20 gekoppelt, der Exspirationsschlauch 42 mit dem Exspirationsventil 22. Von den beiden Ventilen ist jeweils nur eines gleichzeitig zum Durchlass einer Gasströmung geöffnet. Die Betätigungssteuerung der Ventile 20 und 22 erfolgt ebenfalls durch die Steuereinrichtung 18.

Während eines Beatmungszyklus ist zunächst für die Dauer der Inspirationsphase das Exspirationsventil 22 geschlossen und das Inspirationsventil 20 geöffnet, sodass frisches Atemgas vom Gehäuse 14 zum Patienten 12 geleitet werden kann. Eine Strömung des frischen Atemgases wird durch gezielte Druckerhöhung des Atemgases durch die Druckveränderungsanordnung 16 bewirkt. Aufgrund der Druckerhöhung strömt das frische Atemgas in die Lunge des Patienten 12 und expandiert dort den lungennahen Körperbereich, also insbesondere den Brustkorb, gegen die individuelle Elastizität der lungennahen Körperteile. Hierdurch steigt auch der Gasdruck im Inneren der Lunge des Patienten 12 an.

Am Ende der Inspirationsphase wird das Inspirationsventil 20 geschlossen und das Exspirationsventil 22 geöffnet. Es beginnt die Exspirationsphase. Aufgrund des bis zum Ende der Inspirationsphase erhöhten Gasdrucks des in der Lunge des Patienten 12 befindlichen Atemgases strömt dieses nach dem Öffnen des Exspirationsventils 22 in die Atmosphäre, wobei sich mit fortschreitender Strömungsdauer der Gasdruck in der Lunge des Patienten 12 verringert. Erreicht der Gasdruck in der Lunge 12 beispielsweise einen an der Beatmungsvorrichtung 10 eingestellten positiven endexspiratorischen Druck, also einen geringfügig höheren Druck als den Atmosphärendruck, wird die Exspirationsphase mit dem Schließen des Exspirationsventils 22 beendet und es schließt sich ein weiterer Beatmungszyklus an. Dies kann eines von mehreren Triggerkriterien sein, durch deren Vorliegen die Steuereinrichtung eine Umsteuerung des Beatmungsgeräts von exspiratorischem zu inspiratorischem Betrieb triggern kann.

Während der Inspirationsphase wird dem Patienten 12 das sogenannte Beatmungs-Tidalvolumen zugeführt, also das Atemgas-Volumen pro Atemhub. Das Beatmungs-Tidalvolumen multipliziert mit der Anzahl an Beatmungszyklen pro Minute, also multipliziert mit der Beatmungsfrequenz, ergibt das Minutenvolumen der vorliegend durchgeführten künstlichen Beatmung.

Bevorzugt ist die Beatmungsvorrichtung 10, insbesondere die Steuereinrichtung 18, dazu ausgebildet, Beatmungs-Betriebsparameter, die den Beatmungsbetrieb der Beatmungsvorrichtung 10 kennzeichnen, während des Beatmungsbetriebs wiederholt zu aktualisieren bzw. zu ermitteln, um sicherzustellen, dass der Beatmungsbetrieb zu jedem Zeitpunkt möglichst optimal auf den jeweils zu beatmenden Patienten 12 abgestimmt ist. Besonders vorteilhaft erfolgt die Bestimmung eines oder mehrerer Beatmungs-Betriebsparameter mit der Beatmungsfrequenz, sodass für jeden Beatmungszyklus aktuelle und damit optimal an den Patienten 12 angepasste Beatmungs-Betriebsparameter bereitgestellt werden können.

Die Beatmungsvorrichtung 10 ist wenigstens mit einem Flusssensor 44, vorzugsweise mit noch weiteren Sensoren, wie etwa einem Drucksensor zur Messung des Atemgasdrucks in der Atemgas-Leitungsanordnung 30, datenübertragungsmäßig verbunden. Der Flusssensor 44 erfasst die in der Atemgas-Leitungsanordnung 30 herrschende Atemgas-Strömung und gibt ein die die Atemgas-Strömung bzw. den Atemgas-Fluss repräsentierendes Signal aus. Der Flusssensor 44 ist bevorzugt mittels einer Sensor-Leitungsanordnung 46 mit den Dateneingängen 26 der Steuereinrichtung 18 gekoppelt. Die Sensor-Leitungsanordnung 46 kann, muss jedoch nicht, elektrische Signalübertragungsleitungen umfassen. Sie kann ebenso Schlauchleitungen aufweisen, die den in Strömungsrichtung beiderseits des Flusssensors 44 herrschenden Gasdruck an die Dateneingänge 26 übertragen, wo diese von in Figur 1 nicht dargestellten Drucksensoren quantifiziert werden.

Lediglich der Vollständigkeit halber sei darauf hingewiesen, dass die erfindungsgemäße Beatmungsvorrichtung 10 als mobile Beatmungsvorrichtung 10 auf einem rollbaren Gestell 48 aufgenommen sein kann.

Der Flusssensor 44 ist bevorzugt in einem Abschnitt der Atemgas-Leitungsanordnung 30 angeordnet, in welchem er sowohl den inspiratorischen Fluss wie auch den exspiratorischen Fluss erfassen kann. Alternativ oder aus Redundanzgründen auch zusätzlich können im Inspirationsschlauch 32 oder/und im Exspirationsschlauch 42 oder/und im Gehäuse 14 je wenigstens ein weiterer Flusssensor vorgesehen sein.

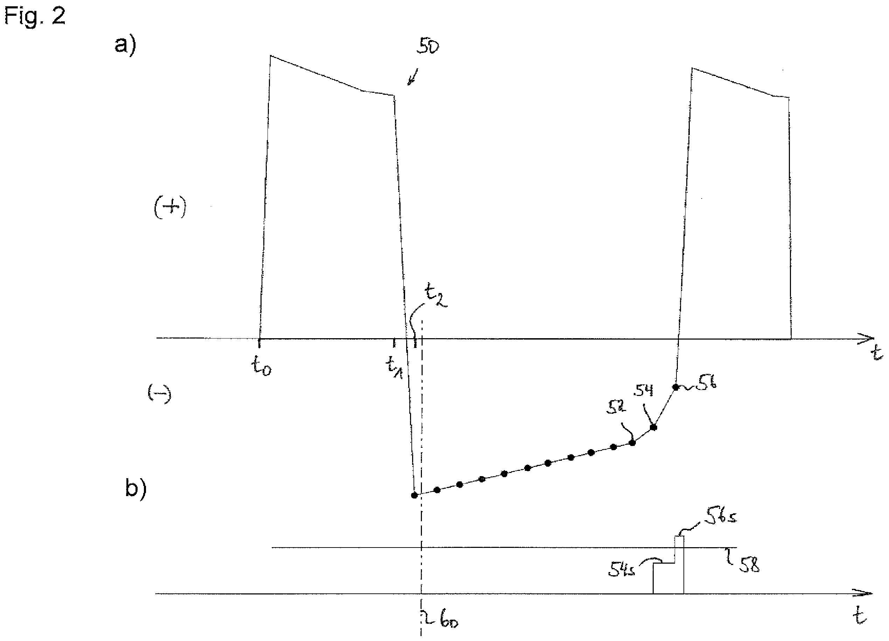

In Figur 2a ist beispielhaft und grob schematisch ein zeitlicher Flusssignalverlauf 50 dargestellt, wie es sich ergibt, wenn der Patient 12 von Figur 1 ein COPD-Patient ist.

Der Inspirationsvorgang beginnt zum Zeitpunkt to, bei welchem die Steuereinrichtung 18 den oben beschriebenen Inspirationsvorgang einleitet und den Druck in der Inspirationsleitung 32 auf einen voreingestellten Inspirationsdruck erhöht.

Durch die Druckerhöhung im Inspirationsschlauch 32 steigt der Atemgasfluss, notiert beispielsweise als Volumenstrom in Volumen pro Zeiteinheit, rasch an und nimmt dann mit zunehmender Füllung der Lunge mit frischem Atemgas betragsmäßig allmählich ab. Der Atemgasfluss zum Patienten 12 hin beziehungsweise in den Patienten 12 hinein sei vereinbarungsgemäß mit positivem Vorzeichen behaftet.

Mit der Füllung der Lunge des Patienten 12 wird dessen Lunge und mit dieser die ihn umgebende Atemmuskulatur und der Brustkorb des Patienten gedehnt. Zu einem Zeitpunkt t₁ findet ein Cycling statt, also ein Umsteuern des Beatmungsgeräts 10 vom inspiratorischen Betrieb in den exspiratorischen Betrieb. Das Inspirationsventil 20 wird geschlossen und das Exspirationsventil 22 wird geöffnet. Hierdurch fällt der Atemgasstrom zum Patienten hin schlagartig ab und kehrt sich in seiner Strömungsrichtung um, getrieben durch die zuvor durch das Einleiten von frischem Atemgas in die Lunge erhöhte Körperspannung im Bereich der Atmungsorgane des Patienten 12. Die Ausatmung des Patienten 12 erfolgt passiv, in dem man die durch das Einblasen von frischem Atemgas gedehnten Körperpartien entspannen lässt.

Durch die Richtungsumkehr wird der Atemgasfluss negativ, wobei erst zum Zeitpunkt t₂ nach dem Umschalten der Ventile: Inspirationsventil 20 und Exspirationsventil 22, sich der eigentliche Exspirationsvorgang einstellt, bei dem das verstoffwechselte Atemgas aus der Lunge des Patienten 12 entweicht. Dies geschieht bei COPD-Patienten aufgrund ihres obstruktiven Atemsystems nur allmählich, sodass der Flusssignalverlauf 50 mit verhältnismäßig geringer und zunächst konstanter Steigung zunimmt. Betragsmäßig nimmt der Atemgasfluss ab, unter Berücksichtigung des Vorzeichens des Flusses nimmt dieser jedoch zu, da er sich hin zu betragsmäßig niedrigeren negativen Werten verändert. Dies bedeutet, dass pro Zeiteinheit das vom COPD-Patienten exspirierte verstoffwechselte Atemgas um einen in etwa konstanten Volumenstrom-Differenzbetrag abnimmt.

Erst mit fortgeschrittener Entleerung der Lunge des COPD-Patienten 12 treten Änderungen, insbesondere Zunahmen, in der Steilheit des Flusssignalverlaufs 50 auf. Hierzu sei auf die drei letzten Stützpunkte des Flusssignalverlaufs, das sind die Stützpunkte 52, 54 und 56 verwiesen.

Wie in Figur 2a erkennbar ist, ändert sich der Betrag der Steigung beziehungsweise der Steilheit des Flusssignalverlaufs 50 zwischen den Stützpunkten 52 und 54 im Vergleich zu den im Stützpunkt 52 vorgehenden Stützpunkten. Die Steigung nimmt zu.

Die Steigung beziehungsweise Steilheit des Flusssignalverlaufs 50 ändert sich erneut zwischen den Stützpunkten 54 und 56. Die Steigung nimmt erneut zu.

In Figur 2b ist über die Zeitachse t, korreliert mit der Zeitachse t von Figur 2a, die Zunahme der Steilheit aufgetragen. Dabei ist, wie es der Darstellung des Flusssignalverlaufs in Figur 2a im Bereich der Stützpunkte 52 bis 56 entspricht, die Änderung der Steilheit des Flusssignalverlaufs 50 vom Stützpunkt 52 zum Stützpunkt 54 im Vergleich zum vorhergehenden Verlauf der Kurve niedriger aufgetragen als die Änderung der Steilheit des Verlaufs 50 zwischen den Stützpunkten 54 und 56. Die Änderungen der Steilheit sind jeweils den zeitlich späteren Stützpunkten eines zur Berechnung der Steilheit herangezogenen Stützpunktepaars zugeordnet, also den Stützpunkten 54 und 56. Der Änderungswert, insbesondere der Zunahmewert, der Steilheit des Flusssignalverlaufs 50 im Bereich der Stützpunkte 52 und 54, verglichen mit einem davor gelegenen Verlaufsabschnitt, ist daher mit 54s bezeichnet und der Änderungswert, insbesondere der Zunahmewert, des Flusssignalverlaufs im Bereich der Stützpunktepaars 54 und 56 relativ zum Abschnitt zwischen dem Stützpunktepaar 50 und 52 ist mit 56s bezeichnet.

In Figur 2b ist außerdem ein Steilheit-Änderungsschwellenwert 58 eingetragen, welcher als Triggerkriterium für das Auslösen eines Übergangs vom exspiratorischen Betrieb zum inspiratorischen Betrieb der Beatmungseinrichtung 10 herangezogen wird.

Der Steilheit-Änderungsschwellenwert 58 ist in Beispiel der Figur 2b für COPD-Patienten zutreffend so gewählt, dass der Änderungswert 54s, bei welchem sich der Patient noch in der Exspirationsphase befindet und keine eigene Anstrengung zur Inspiration unternommen hat, unterhalb des Steilheit-Änderungsschwellenwert 58 gelegen ist.

Der Änderungswert 56s geht allerdings auf einen für COPD-Patienten typischen Wert bei Ausübung einer eigenen Inspirationsanstrengung zurück, sodass dieser Wert den Steilheit-Änderungsschwellenwert 58 überschreitet und somit die Steuereinrichtung 18 veranlasst, das Beatmungsgerät 10 von einem exspiratorischen Betrieb in einen inspiratorischen Betrieb umzuschalten.

Wie in Figur 2b zu erkennen ist, tritt eine massive Zunahme der Steilheit des Flusssignalverlaufs 50 in der Anfangsphase der Exspiration ein, nämlich zum Zeitpunkt t₂, wenn nach deinem ersten betragsmäßig starken Anstieg des Exspirationsflusses der Atemgasfluss aus dem Patienten heraus allmählich betragsmäßig wieder abnimmt.

Um zu verhindern, dass diese Änderung der Steilheit des Flusssignalverlaufs 50 bereits zu Beginn des exspiratorischen Betriebs einen Übergang zum inspiratorischen Betrieb auslöst, ist in der Steuereinrichtung 18 eine zeitliche Schwelle 60 hinterlegt, ab welcher erst eine Änderung der Steilheit des zeitlichen Flusssignalverlaufs 50 zur Bereitstellung eines Triggersignals beobachtet wird. Die zeitliche Schwelle 60 kann beispielsweise eine vorbestimmte Zeitspanne ab dem Schließen des Inspirationsventils 20 oder ab dem Öffnen des Exspirationsventils 22 sein.

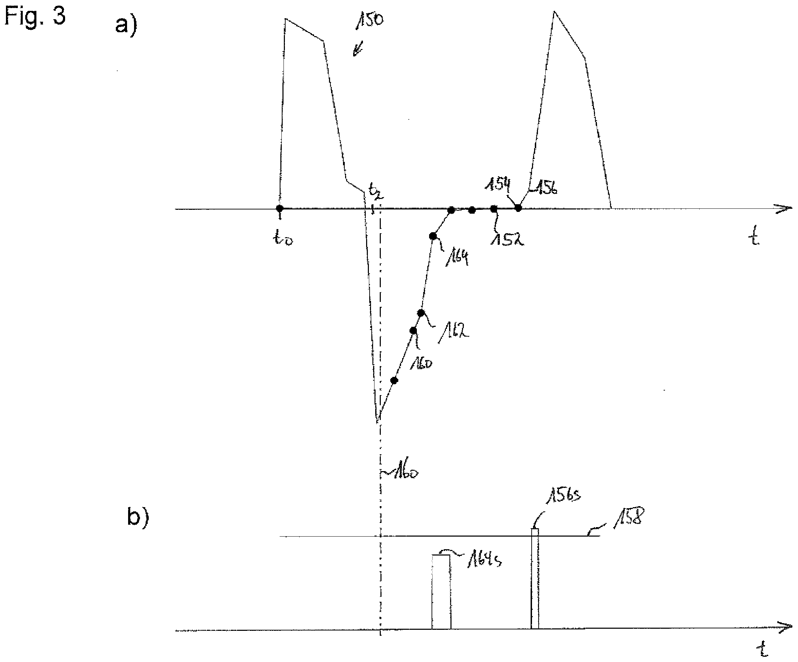

In den Figuren 3a und 3b ist eine den Figuren 2a und 2b jeweils entsprechende Darstellung eines Flusssignalverlaufs 150 für einen ARDS-Patienten dargestellt. ARDS-Patienten haben ein sogenanntes restruktives Atemsystem, dessen Compliance sehr niedrig ist. Das Atemsystem ist weniger elastisch als bei respiratorisch gesunden Patienten. Bei COPD-Patienten ist dagegen die Resistance des Atemsystems gegenüber respiratorisch gesunden Patienten erhöht.

Aufgrund der verringerten Compliance des Atemsystems von ARDS-Patienten erfordert das Einleiten von frischem Atemgas in die Lunge des Patienten eine erhöhte Arbeit gegen die verhältnismäßig unelastische Lunge und die ebenso unelastischen Atemwege. Der Flusssignalverlauf 150 ist daher grundsätzlich steiler als bei respiratorisch gesunden Patienten und vor allem steiler als COPD-Patienten. Die erreichten Maximalbeträge des Flusses sind auch größer.

Gleiche und funktionsgleiche Details wie in den Figuren 2a und 2b sind in den Figuren 3a und 3b mit gleichen Bezugszeichen bezeichnet, jedoch erhöht um die Zahl 100.

Wie in Figur 3a zu erkennen ist, erreicht der exspiratorische Atemgasfluss zu Beginn der Exspirationsphase einen betragsmäßig höheren Wert als im Falle eines COPD-Patienten, was an der obstruktiven Atmung des COPD-Patienten ebenso liegt, wie an der niedrigen Compliance des ARDS-Patienten. Aus im Wesentlichen den gleichen Gründen fällt der exspiratorische Atemgasfluss beim ARDS-Patienten gemäß Figur 3a schneller ab als beim COPD-Patienten.

Dies wird auch dadurch deutlich, dass die exspiratorische Zeitkonstante eines ARDS-Patienten einen erheblich niedrigeren Wert aufweist als jene des COPD-Patienten.

Während beim COPD-Patienten der Flusssignalverlauf während der Exspirationsphase über einen langen Zeitraum mit konstanter Steigung verläuft, ändert sich die Steigung des Flusssignalverlaufs beim ARDS-Patienten üblicherweise während der Exspirationsphase mehrmals.

Für die Auswertung des exspiratorischen Flusssignalverlaufs hinsichtlich des Vorliegens des die Änderung der Steilheit des Flusssignalverlaufs berücksichtigenden Triggerkriteriums werden für das vorliegend diskutierte Triggerkriterium bevorzugt nur jene Änderungen der Steilheit des Flusssignalverlaufs 150 in Betracht gezogen, bei welchen die Steigung des Flusssignalverlaufs 150 zunimmt, bei welchem also die Krümmung des Flusssignalverlaufs 150 bei Betrachtung aus dem positiv Unendlichen der Ordinate eine konkave Gestalt aufweist, während konvexe Änderungen der Steilheit, bei welchen der Flusssignalverlauf 150 flacher wird, gar nicht erst in Betracht gezogen werden.

Dementsprechend sind in Figur 3b nur jene Änderungen der Steigung der Flusssignalkurve 150 aufgetragen, die positiv sind.

Zu erkennen ist, dass eine verhältnismäßig deutliche Zunahme der Steigung während des Exspirationsvorgangs bei Stützpunkt 162 einen Wert erreicht, welcher für COPD-Patienten weit jenseits des in Figur 2b eingezeichneten Steilheit-Änderungsschwellenwerts 58 liegen und somit einen Übergang von exspiratorischem zu inspiratorischem Betrieb auslösen würde.

Für ARDS-Patienten wäre eine Umsteuerung des Beatmungsgeräts 10 in diesem Abschnitt der Exspirationsphase sehr unangenehm, da der Patient mitten in einem Exspirationsvorgang mit einer ihm entgegenströmenden Inspirationsströmung belastet würde.

Der Steilheit-Änderungsschwellenwert 158 für ARDS-Patienten ist daher vorteilhafterweise größer gewählt als jener für COPD-Patienten. Ein entsprechender Steilheit-Änderungsschwellenwert für respiratorisch gesunde Patienten könnte zwischen den Schwellenwerten 58 und 158 liegen.

Am Ende des Exspirationsvorgangs, ab dem Stützpunkt 154 beginnt der im PSV-Modus unterstützend beatmete ARDS-Patient mit dem Ansaugen von Atemgas, wodurch es zu einer erheblichen Zunahme der Steilheit des Flusssignalverlaufs 150 kommt. Der Verlaufsabschnitt zwischen den Stützpunkten 154 und 156 ist erheblich steiler beziehungsweise weist eine erheblich größere Steigung auf als der unmittelbar vorhergehende Verlaufsabschnitt zwischen den Stützpunkten 152 und 154. Aufgrund der eigenen Inspirationsanstrengung des während der Beatmung wenigstens gelegentlich spontan atmenden ARDS-Patienten findet dieser Anstieg im Flusssignalverlauf bis hinein zu einem positiven Flusssignal statt. Der zugehörige Änderungswert 156s ist in Figur 3b dargestellt. Er überschreitet den für ARDS-Patienten definierten Steilheit-Änderungsschwellenwert 158 und löst damit ein Umsteuern von exspiratorischem zu inspiratorischem Betrieb des Beatmungsgeräts 10 aus.

Ein Datenspeicher des Beatmungsgeräts 10 kann in Form eines Kennfelds, einer Tabelle, einer hinterlegten Funktion oder Näherungsfunktion und dergleichen einen Zusammenhang zwischen dem in einem Beatmungsfall anzuwendenden Steilheit-Änderungsschwellenwert und einer den Zustand des Atemsystems des Patienten 12 charakterisierenden exspiratorischen Zeitkonstante enthalten.

Die Steuereinrichtung 18 kann dann ausgehend von der exspiratorischen Zeitkonstante des aktuell zu beatmenden Patienten 12 anhand des hinterlegten Zusammenhangs den für den aktuell zu beatmenden Patienten 12 anzuwendenden Steilheit-Änderungsschwellenwert abfragen oder durch Rechenoperationen ermitteln.

Die exspiratorische Zeitkonstante des aktuell zu beatmenden Patienten 12 kann von ärztlichem Personal in das Beatmungsgerät 10 manuell eingegeben werden, wenn diese bekannt ist, etwa um wenigstens einen Anfangswert für den Steilheit-Änderungsschwellenwert zu haben.

In vorteilhafter Weise ist die Steuereinrichtung 18 dazu ausgebildet, anhand der ihr zugänglichen Sensorinformation die exspiratorische Zeitkonstante des jeweils zu beatmenden Patienten 12 zu ermitteln. Hierfür sind im Stand der Technik mehrere Maßnahmen bekannt. Eine mögliche Art und Weise zur Ermittlung der exspiratorischen Zeitkonstante kann allein aufgrund der vom Flusssensor 44 erhaltenen Information ausgeführt werden. Beispielsweise kann die exspiratorische Zeitkonstante anhand des während einer Exspirationsphase ausgeatmeten Atemgasvolumens und dem dabei während derselben Exspirationsphase aufgetretenen maximalen Atemgasfluss ermittelt werden. Auf weitere mögliche Ermittlungsverfahren zur Ermittlung der exspiratorischen Zeitkonstante wurde oben in der Beschreibungseinleitung bereits hingewiesen.

Mit dem vorliegenden erfindungsgemäßen Beatmungsgerät 10 können somit selbst Patienten mit diametral unterschiedlichen pathologischen Atemsystemen mit hoher Synchronität schonend und zuverlässig beatmet werden, da das Beatmungsgerät 10 in der Lage ist, den Zustand des Atemsystems des Patienten 12 zu ermitteln und ausgehend von dem ermittelten Zustand des Atemsystems das steilheitsabhängige Triggerkriterium für eine Umsteuerung des Beatmungsgeräts 10 von exspiratorischem zu inspiratorischem Betrieb an den jeweils zu beatmenden Patienten anzupassen.

## Patentansprüche

1. Beatmungsgerät (10) zur wenigstens unterstützenden Beatmung von im gesunden Zustand atmenden Lebewesen (12), wobei das Beatmungsgerät umfasst:
- eine Anschlussformation zur Verbindung mit einem Atemgasvorrat,
- eine bei inspiratorischem Betrieb des Beatmungsgeräts (10) frisches inspiratorisches Atemgas von der Anschlussformation zu einer Patientenschnittstelle (31) hin führende und bei exspiratorischem Betrieb verstoffwechseltes exspiratorisches Atemgas von der Patientenschnittstelle (31) weg führende Atemgas-Leitungsanordnung (30),
- eine Druckveränderungsanordnung (16) zur Veränderung des Drucks von Atemgas in der Atemgas-Leitungsanordnung (30),
- einen Flusssensor (44), welcher dazu ausgebildet und angeordnet ist, ein einen Atemgas-Volumenstrom wenigstens von exspiratorischem Atemgas in der Atemgas-Leitungsanordnung (30) repräsentierendes Flusssignal (50; 150) bereitzustellen,
- eine den Betrieb des Beatmungsgeräts (10) steuernde Steuereinrichtung (18), welche dazu ausgebildet ist, einen Übergang von exspiratorischem Betrieb zu inspiratorischem Betrieb des Beatmungsgeräts (10) dann zu triggern, wenn eine Zunahme der Steilheit eines Flusssignalverlaufs (50; 150), welcher zeitlich nacheinander bereitgestellte Flusssignale wiedergibt, einen Steilheit-Änderungsschwellenwert (58; 158) übersteigt,
**dadurch gekennzeichnet, dass** die Steuereinrichtung (18) weiter dazu ausgebildet ist, den Steilheit-Änderungsschwellenwert (58; 158) abhängig von einer exspiratorischen Zeitkonstante des jeweils zu beatmenden Patienten (12) zu ermitteln und den Übergang von exspiratorischem Betrieb zu inspiratorischem Betrieb des Beatmungsgeräts (10) dann zu triggern, wenn die Zunahme der Steilheit des Flusssignalverlaufs (50; 150) den nach Maßgabe der exspiratorischen Zeitkonstante ermittelten Steilheit-Änderungsschwellenwert (58; 158) übersteigt.

2. Beatmungsgerät (10) nach Anspruch 1,
**dadurch gekennzeichnet, dass** die Steuereinrichtung (18) dazu ausgebildet ist, den Betrag des Steilheit-Änderungsschwellenwerts (58; 158) mit zunehmender exspiratorischer Zeitkonstante zu verringern und umgekehrt.

3. Beatmungsgerät (10) nach Anspruch 1 oder 2,
**dadurch gekennzeichnet, dass** die Steuereinrichtung (18) dazu ausgebildet ist, die exspiratorische Zeitkonstante in einer jeweiligen Beatmungsanwendung zu ermitteln.

4. Beatmungsgerät (10) nach Anspruch 3,
**dadurch gekennzeichnet, dass** die Steuereinrichtung (18) dazu ausgebildet ist, die exspiratorische Zeitkonstante atemzugsweise zu ermitteln.

5. Beatmungsgerät (10) nach Anspruch 3 oder 4,
**dadurch gekennzeichnet, dass** die Steuereinrichtung (18) dazu ausgebildet ist, die exspiratorische Zeitkonstante nach Maßgabe des Verhältnisses von ausgeatmetem Volumen und bei diesem Exspirationsvorgang aufgetretenem maximalen exspiratorischen Volumenstrom, bevorzugt iterativ numerisch, oder/und nach Maßgabe der bei einem Atemhub auftretenden Resistance und Compliance zu ermitteln.

6. Beatmungsgerät (10) nach einem der vorhergehenden Ansprüche,
**dadurch gekennzeichnet, dass** es einen von der Steuereinrichtung (18) wenigstens lesbaren Datenspeicher aufweist, in welchem ein Zusammenhang zwischen dem Betrag des Steilheit-Änderungsschwellenwerts (58; 158) und der exspiratorischen Zeitkonstante oder ein Zusammenhang zwischen einem Änderungswerts des Betrags des Steilheit-Änderungsschwellenwerts (58; 158) und der exspiratorischen Zeitkonstante hinterlegt ist.

7. Beatmungsgerät (10) nach Anspruch 6,
**dadurch gekennzeichnet, dass** im Datenspeicher ein funktioneller Zusammenhang zwischen dem Betrag des Steilheit-Änderungsschwellenwerts (58; 158) und der exspiratorischen Zeitkonstante oder ein funktioneller Zusammenhang zwischen einem Änderungswerts des Betrags des Steilheit-Änderungsschwellenwerts (58; 158) und der exspiratorischen Zeitkonstante hinterlegt ist und dass die Steuereinrichtung (18) dazu ausgebildet ist, einen anzuwendenden Steilheit-Änderungsschwellenwert (58; 158) ausgehend von der exspiratorischen Zeitkonstante anhand des funktionellen Zusammenhangs zu ermitteln.

8. Beatmungsgerät (10) nach einem der vorhergehenden Ansprüche,
**dadurch gekennzeichnet, dass** die Steuereinrichtung (18) dazu ausgebildet ist, das vom Flusssensor (44) bereitgestellte Flusssignal (50; 150) zu glätten, etwa durch Tiefpassfilterung oder durch gleitende Mittelwertbildung.

9. Beatmungsgerät (10) nach einem der vorhergehenden Ansprüche,
**dadurch gekennzeichnet, dass** die Steuereinrichtung (18) dazu ausgebildet ist, den Übergang von exspiratorischem Betrieb zu inspiratorischem Betrieb des Beatmungsgeräts (10) nicht vor Ablauf einer vorbestimmten Zeitspanne (60; 160) nach einem vorgegebenen charakteristischen Ereignis, wie etwa ein Beginn des exspiratorischen Betriebs oder ein Basisniveaudurchgang des Flusssignals (50; 150), zu triggern.

10. Beatmungsgerät (10) nach Anspruch 9,
**dadurch gekennzeichnet, dass** das Steuergerät dazu ausgebildet ist, die vorbestimmte Zeitspanne (60; 160) nach Maßgabe der exspiratorischen Zeitkonstante zu ermitteln.

## Claims

1. A ventilator (10) for at least supportive ventilation of living beings (12) breathing in a healthy state, the ventilator encompassing:
- a connector configuration for connection to a respiratory gas reservoir;
- a respiratory gas conduit arrangement (30) conveying fresh inspiratory respiratory gas from the connector configuration to a patient interface (31) upon inspiratory operation of the ventilator (10), and conveying metabolized expiratory respiratory gas away from the patient interface (31) upon expiratory operation;
- a pressure modifying arrangement (16) for modifying the pressure of respiratory gas in the respiratory gas conduit arrangement (30);
- a flow sensor (44) that is embodied and arranged to furnish a flow signal (50; 150) representing a respiratory gas volumetric flow at least of expiratory respiratory gas in the respiratory gas conduit arrangement (30);
- a control device (18) that controls the operation of the ventilator (10) and is embodied to trigger a transition from expiratory operation to inspiratory operation of the ventilator (10) when an increase in the slope of a flow signal profile (50; 150), which reproduces flow signals furnished successively in time, exceeds a slope change threshold value (58; 158),
wherein the control device (18) is further embodied to ascertain the slope change threshold value (58; 158) as a function of an expiratory time constant of the respective patient (12) to be ventilated, and to trigger the transition from expiratory operation to inspiratory operation of the ventilator (10) when the increase in the slope of the flow signal profile (50; 150) exceeds the slope change threshold value (58; 158) ascertained in accordance with the expiratory time constant.

2. The ventilator (10) according to Claim 1, wherein the control device (18) is embodied to decrease the magnitude of the slope change threshold value (58; 158) with an increasing expiratory time constant, and vice versa.

3. The ventilator (10) according to Claim 1 or 2, wherein the control device (18) is embodied to ascertain the expiratory time constant in one respective ventilation use instance.

4. The ventilator (10) according to Claim 3, wherein the control device (18) is embodied to ascertain the expiratory time constant breath by breath.

5. The ventilator (10) according to Claim 3 or 4, wherein the control device (18) is embodied to ascertain the expiratory time constant, preferably iteratively numerically, in accordance with the ratio between exhaled volume and the maximum expiratory volumetric flow that occurred in the context of that expiration event, and/or in accordance with the resistance and compliance that occur in the context of one breath.

6. The ventilator (10) according to one of the preceding claims, wherein it comprises a data memory, at least readable by the control device (18), in which a correlation between the magnitude of the slope change threshold value (58; 158) and the expiratory time constant, or a correlation between a change value of the magnitude of the slope change threshold value (58; 158) and the expiratory time constant, is stored.

7. The ventilator (10) according to Claim 6, wherein a functional correlation between the magnitude of the slope change threshold value (58; 158) and the expiratory time constant, or a functional correlation between a change value of the magnitude of the slope change threshold value (58; 158) and the expiratory time constant, is stored in the data memory; and the control device (18) is embodied to ascertain, proceeding from the expiratory time constant based on the functional correlation, a slope change threshold value (58; 158) that is to be used.

8. The ventilator (10) according to one of the preceding claims, wherein the control device (18) is embodied to smooth the flow signal (50; 150) furnished by the flow sensor (44), for example by low-pass filtering or by calculation of a moving average.

9. The ventilator (10) according to one of the preceding claims, wherein the control device (18) is embodied not to trigger the transition from expiratory operation to inspiratory operation of the ventilator (10) until a predetermined time span (60; 160) has elapsed after a predefined characteristic event, for example a beginning of expiratory operation or a baseline-level crossing of the flow signal (50; 150).

10. The ventilator (10) according to Claim 9, wherein the control device is embodied to ascertain the predetermined time span (60; 160) in accordance with the expiratory time constant.

## Revendications

1. Appareil respiratoire (10) au moins pour la respiration assistée d'êtres vivants (12) respirant en bonne santé, l'appareil respiratoire comprenant :
- une formation de connexion pour la communication avec un réservoir de gaz respiratoire,
- un arrangement de conduite de gaz respiratoire (30) transportant pendant l'opération inspiratoire de l'appareil respiratoire (10) du gaz inspiratoire frais de la formation de connexion à une interface patient (31) et éloignant pendant l'opération expiratoire du gaz expiratoire métabolisé de l'interface patient (31),
- un arrangement de modification de pression (16) pour modifier la pression de gaz respiratoire dans l'arrangement de conduite de gaz respiratoire (30),
- un capteur de débit (44) adapté et arrangé pour prévoir un signal de débit (50 ; 150) représentant un débit volumétrique au moins de gaz expiratoire dans l'arrangement de conduite de gaz respiratoire (30),
- un dispositif de contrôle (18) réglant l'opération de l'appareil respiratoire (10), adapté pour déclencher une transition de l'opération expiratoire de l'appareil respiratoire (10) à l'opération inspiratoire de l'appareil respiratoire (10) lorsqu'une augmentation d'une pente du développement du signal de débit (50 ; 150) qui représente des signaux de débit prévus successivement dans le temps dépasse une valeur limite de modification de pente (58 ; 158),
**caractérisé en ce que** le dispositif de contrôle (18) est en outre adapté pour déterminer la valeur limite de modification de pente (58 ; 158) en fonction d'une constante de temps expiratoire du patient respectif à ventiler (12) et pour déclencher la transition de l'opération expiratoire de l'appareil respiratoire (10) à l'opération inspiratoire lorsque l'augmentation de la pente du développement du signal de débit (50 ; 150) dépasse la valeur limite de modification de pente (58 ; 158) déterminée selon la constante de temps expiratoire.

2. Appareil respiratoire (10) selon la revendication 1,
**caractérisé en ce que** le dispositif de contrôle (18) est adapté pour réduire le montant de la valeur limite de modification de pente (58 ; 158) avec l'augmentation de la constante de temps expiratoire et vice versa.

3. Appareil respiratoire (10) selon les revendications 1 ou 2,
**caractérisé en ce que** le dispositif de contrôle (18) est adapté pour déterminer la constante de temps expiratoire dans une application de ventilation correspondante.

4. Appareil respiratoire (10) selon la revendication 3,
**caractérisé en ce que** le dispositif de contrôle (18) est adapté pour déterminer la constante de temps expiratoire par souffle.

5. Appareil respiratoire (10) selon la revendication 3 ou 4,
**caractérisé en ce que** le dispositif de contrôle (18) est adapté pour déterminer la constante de temps expiratoire en fonction du rapport entre le volume expiré et le débit volumique maximale observé pendant cette opération d'expiration, de préférence de manière itérative numérique, ou/et en fonction de la résistance et de la conformité observées pendant une phase respiratoire.

6. Appareil respiratoire (10) selon une des revendications précédentes, **caractérisé en ce qu'**il comprend une mémoire de données au moins lisible par le dispositif de contrôle (18) où une relation est notée entre la valeur limite de modification de pente (58 ; 158) et la constante de temps expiratoire ou une relation entre une valeur de modification de la valeur limite de modification de pente (58 ; 158) et la constante de temps expiratoire.

7. Appareil respiratoire (10) selon la revendication 6,
**caractérisé en ce qu'**on trouve dans la mémoire de données une relation fonctionnelle entre la valeur limite de modification de pente (58 ; 158) et la constante de temps expiratoire ou une relation fonctionnelle entre une valeur de modification de la valeur limite de modification de pente (58 ; 158) et la constante de temps expiratoire et **en ce que** le dispositif de contrôle (18) est adapté pour déterminer une valeur limite de modification de pente (58 ; 158) à appliquer sur la base de la constante de temps expiratoire et de la relation fonctionnelle.

8. Appareil respiratoire (10) selon une des revendications précédentes, **caractérisé en ce que** le dispositif de contrôle (18) est adapté pour lisser le signal de débit (50 ; 150) prévu par le capteur de débit (44), par exemple par un filtrage passe-bas ou par une moyenne glissante.

9. Appareil respiratoire (10) selon une des revendications précédentes, **caractérisé en ce que** le dispositif de contrôle (18) est adapté pour ne pas déclencher la transition de l'opération expiratoire à l'opération inspiratoire de l'appareil respiratoire (10) avant l'issue d'un laps de temps prédéterminé (60 ; 160) après un évènement caractéristique prédéterminé, comme par exemple un début de l'opération expiratoire ou un processus de niveau de base du signal de débit (50 ; 150).

10. Appareil respiratoire (10) selon la revendication 9,
**caractérisé en ce que** le dispositif de contrôle est adapté pour déterminer le laps de temps prédéterminé (60 ; 160) en fonction de la constante de temps expiratoire.
